# EUROPEAN PATENT APPLICATION

(11) **EP 4 592 388 A1**
(43) Date of publication of application: **30.07.2025**
(21) Application number: 23885883.1
(22) Date of filing: 02.11.2023
(51) Int. Cl.: C12N 15/11, C07K 2/00, C12N 1/20, C12Q 1/02, C12Q 1/68, G01N 33/50

(54) **BIOMARKER FOR USE IN PREDICTION OF OCCURRENCE OF ACNE VULGARIS, PREDICTION OF SEVERITY AND DETERMINATION OF SEVERITY**

(30) Priority: 04.11.2022 US 202263422489 P
(71) Applicant: The University of Osaka, Suita-shi Osaka 565-0871 (JP); Rohto Pharmaceutical Co., Ltd., Osaka-shi, Osaka 544-8666 (JP)
(72) Inventor: MATSUOKA, Yumi, Suita-shi, Osaka 565-0871 (JP); FUJIMOTO, Manabu, Suita-shi, Osaka 565-0871 (JP); SUGIHIRA, Takashi, Osaka-shi, Osaka 544-8666 (JP)
(74) Representative: Mewburn Ellis LLP
(86) International application number: PCT/JP2023/039756
(87) International publication number: WO 2024/096129

(57) **Abstract**

Disclosed is a biomarker for use in the prediction of the occurrence and the severity of acne vulgaris, and in the determination of the severity of acne vulgaris.

## Description

### TECHNICAL FIELD

The present invention relates to a biomarker for use in predicting the occurrence of acne vulgaris and determining the severity of acne vulgaris.

### BACKGROUND ART

Acne vulgaris is a skin disease with the eighth largest number of affected people among all diseases (Non-Patent Document 1). Acne vulgaris is a very common skin disease that affects about 85% of teenagers, and since the disease occurs frequently on the face as the site of occurrence, it is directly related to appearance, and for this reason, the disease has a significant impact on mental health (Non-Patent Documents 2 and 3). Acne vulgaris is a multifactorial inflammatory skin disease, and it has been reported that the skin microbiome plays a part in the pathogenesis (Non-Patent Document 4).

Various antimicrobial drugs are currently used as medicines for acne vulgaris. Benzoyl peroxide in particular has attracted attention as an antimicrobial drug that causes less occurrence of resistant bacteria; however, in the first place, dysbiosis of the microbiome occurs in the skin of targets who have been administered with an antimicrobial drug, and the stage of a fundamental treatment that completely cures acne vulgaris (that is, a treatment that alleviates the symptoms of acne vulgaris while suppressing dysbiosis) has not yet been reached.

### CITATION LIST

### NON-PATENT DOCUMENTS

Non-Patent Document 1: Sara Moradi Tuchayi et al., "Acne vulgaris", Nat Rev Dis Primers 17:1:15029 (2015).

Non-Patent Document 2: Mallon E et al., "The quality of life in acne: a comparison with general medical conditions using generic questionnaires", Br J Dermatol. 140(4):672-6 (1999).

Non-Patent Document 3: Stamu-O'Brien C et al., "Psychodermatology of acne: Psychological aspects and effects of acne vulgaris", J Cosmet Dermatol 20(4):1080-1083 (2021).

Non-Patent Document 4: Williams HC, Dellavalle RP, Garner S, "Acne vulgaris", Lancet 379(9813):361-72 (2012).

### Summary of Invention

### Technical Problem

In order to aim for a complete cure for acne vulgaris, it is considered effective to elucidate the pathological mechanism thereof and discover specific targets. It is an object of the present disclosure to provide a biomarker for use in predicting the occurrence, predicting the aggravation, and/or determining the severity of acne vulgaris, and a screening method for a skin microbiome-improving substance, a skin microbiome-improving composition, and a substance or a composition for treating, improving, alleviating, or preventing an inflammatory skin disease (for example, acne vulgaris), the method including examining changes in the biomarker.

### Solution to Problem

In order to solve the above-described problems, the inventors of the present invention conducted human transcriptome analysis, microbiome analysis, and microbial flora metatranscriptome analysis using samples derived from severe acne patients, non-severe acne patients, and healthy individuals. As a result, human genes that showed significantly (FDR < 0.05) high values in patients with high-severity acne vulgaris and a significant positive correlation with severity (Rs > 0.4, p < 0.05) were found. In addition, microbiomes that showed significant differences between severely ill patients (number of inflammatory skin rashes ≥ 5) and non-severely ill patients (number of inflammatory skin rashes < 5) were found. Furthermore, the present inventors clarified pathways derived from Propionibacterium (Cutibacterium) acnes and Staphylococcus epidermidis, which are positively correlated (Rs > 0.4, p < 0.05) with the severity of acne vulgaris (number of inflammatory skin rashes) and with the quantity parameter of the quantity of fatty acids that has been suggested to be related to the severity of acnes vulgaris. Based on these findings, the present inventors developed a biomarker for use in predicting the occurrence, predicting the aggravation, or determining the severity of acne vulgaris, and a screening method for a skin microbiome-improving substance and a skin microbiome-improving composition.

The present disclosure relates to, for example, the following.
[1] A biomarker for use in predicting the occurrence, predicting the aggravation, and/or determining the severity of acne vulgaris, the biomarker being at least one selected from the group consisting of the following (I) to (III):
   (I) at least one human gene selected from the group consisting of IL10, RBP4, CXCL8, NOD2, CYP11B1, CCR1, GSTM1, MEN1, DLK1, DPF2, SLC52A2, KRT17, SEC24C, MSX2, ISCU, DPAGT1, LCT, SAG, LFNG, PAPOLG, IL18BP, HOXA13, VIP, MC5R, B3GNT2, CSK, FGF6, AP5B1, and IL17D, and a biomolecule encoded by the at least one human gene;
   (II) at least one bacterium selected from the group consisting of Staphylococcus sp., Pseudomonas sp., Lactobacillus sp., Peptoniphilus sp., Brevundimonas sp., Leptotrichia sp., Prevotella sp., Corynebacterium sp., Dialister sp., Capnocytophaga sp., Brevibacterium sp., Massilia sp., Sphingomonas sp., Streptococcus sp., and Lachnospiraceae sp., and a nucleic acid derived from the at least one bacterium; and
   (III) at least one pathway selected from the group consisting of pathways of the following derived from Propionibacterium acnes:
      PWY-7220: adenosine deoxyribonucleotides de novo biosynthesis II,
      PWY-7222: guanosine deoxyribonucleotides de novo biosynthesis II,
      PWY-7208: superpathway of pyrimidine nucleobases salvage,
      PWY-7219: adenosine ribonucleotides de novo biosynthesis,
      PWY-7220: adenosine deoxyribonucleotides de novo biosynthesis II,
      PWY-7222: guanosine deoxyribonucleotides de novo biosynthesis II,
      PWY-7228: superpathway of guanosine nucleotides de novo biosynthesis I,
      PWY-6609: adenine and adenosine salvage III,
      PWY-6897 thiamin salvage II,
      PWY-7221: guanosine ribonucleotides de novo biosynthesis,
      DAPLYSINESYN-PWY: L-lysine biosynthesis I,
      PWY-3841: folate transformations II,
      PWY-2942: L-lysine biosynthesis III, and
      COA-PWY: coenzyme A biosynthesis I, and
      pathways of the following derived from Staphylococcus epidermidis:
         PWY-5100: pyruvate fermentation to acetate and lactate II,
         VALSYN-PWY: L-valine biosynthesis, and
         PWY-7111: pyruvate fermentation to isobutanol (engineered),
         at least one biomolecule constituting the at least one pathway, and a nucleic acid encoding the biomolecule.
[2] The biomarker according to [1], wherein a subject for the biomarker is a human.
[3] The biomarker according to [1] or [2], wherein the biomarker is contained in a swab sample collected from a subject.
[4] The biomarker according to [3], wherein the swab sample is collected from the skin.
[5] The biomarker according to any one of [1] to [4], wherein an increase in an amount of the biomarker indicates an increase in severity (worsening of symptoms) of acne vulgaris in a subject.
[6] The biomarker according to any one of [1] to [4], wherein a decrease in an amount of the biomarker indicates a decrease in severity (improvement of symptoms) of acne vulgaris in a subject.
[7] A method for determining severity of acne vulgaris in a subject, the method including:
   measuring the biomarker according to any one of [1] to [6] in a sample derived from the subject.
[8] A method for assisting in determination of severity of acne vulgaris in a subject, the method including:
   measuring the biomarker according to any one of [1] to [6] in a sample derived from the subject.
[9] A method for collecting data for determining severity of acne vulgaris in a subject, the method including:
   measuring the biomarker according to any one of [1] to [6] in a sample derived from the subject.
[10] A method for predicting likelihood of occurrence or likelihood of aggravation of acne vulgaris in a subject, the method including:
   measuring the biomarker according to any one of [1] to [6] in a sample derived from the subject.
[11] A method for assisting in prediction of likelihood of occurrence or likelihood of aggravation of acne vulgaris in a subject, the method including:
   measuring the biomarker according to any one of [1] to [6] in a sample derived from the subject.
[12] A method for collecting data for predicting likelihood of occurrence or likelihood of aggravation of acne vulgaris in a subject, the method including:
   measuring the biomarker according to any one of [1] to [6] in a sample derived from the subject.
[13] A screening method for a skin microbiome-improving substance or a skin microbiome-improving composition, the method including:
   measuring the biomarker according to any one of [1] to [6] in a sample derived from the subject.
[14] A screening method for a skin microbiome-improving substance or a skin microbiome-improving composition, the method including:
   measuring an amount of the biomarker according to any one of [1] to [6] in samples derived from a subject and collected before administration and after administration of a test substance or a test composition to the subject.
[15] A screening method for a substance or a composition for treating, improving, alleviating, or preventing an inflammatory skin disease, the method including:
   measuring the biomarker according to any one of [1] to [6] in a sample derived from the subject.
[16] A screening method for a substance or a composition for treating, improving, alleviating, or preventing an inflammatory skin disease, the method including:
   measuring an amount of the biomarker according to any one of [1] to [6] in samples derived from a subject and collected before administration and after administration of a test substance or a test composition to the subject.
[17] The screening method for according to [15] or [16], wherein the inflammatory skin disease is acne vulgaris.
[18] The screening method for according to any one of [13] to [17], further including the following steps in the following order:
   collecting a sample from a subject;
   administering a test substance or a test composition to the subject; and
   collecting a sample from the subject.

### Advantageous Effects of Invention

According to the present disclosure, a biomarker for use in predicting the occurrence, predicting the aggravation, and/or determining the severity of acne vulgaris. Since the level of the biomarker of the present disclosure increases with the severity of acne vulgaris, the biomarker serves as a physiological indicator indicating acne progression.

Furthermore, according to the present disclosure, there are provided methods related to the determination of the severity of acne vulgaris in a subject, or to the prediction of the likelihood of aggravation of acne vulgaris in a subject, the methods including measuring a biomarker for use in predicting the occurrence, predicting the aggravation, and/or determining the severity of acne vulgaris. According to these methods, the severity of acne vulgaris can be determined, or the aggravation of acne vulgaris can be predicted, by measuring a factor that varies in a subject in a stage in which acne has formed or is in a forming stage, that is, a biomarker related to acne vulgaris of the present invention.

According to the present disclosure, there is disclosed a screening method for a skin microbiome-improving substance or a skin microbiome-improving composition, the method including measuring a biomarker for use in predicting the occurrence, predicting the aggravation, and/or determining the severity of acne vulgaris. The skin microbiome-improving substance, the skin microbiome-improving composition, or a candidate thereof selected in this manner can be utilized as an active ingredient or a candidate thereof for a therapeutic agent, an improving agent, an alleviating agent, or a prophylactic agent for inflammatory skin diseases including acne vulgaris.

### Description of Embodiments

Embodiments of the present disclosure will be described below; however, the present disclosure is not limited to the following embodiments.

### [Biomarker]

In the present disclosure, the term "biomarker" means a substance whose presence or absence of the expression and/or whose change in the amount occurs during the development and aggravation (progression) of a certain disease and/or previous stages thereof. Since a biomarker is a factor that undergoes variation during the development and aggravation of a certain disease, and/or previous stages thereof, it is likely that the biomarker may be used for the determination of the development and aggravation of the disease.

In an aspect, the biomarker according to the present embodiment may be a biomarker for use in predicting the occurrence, predicting the aggravation, and/or determining the severity of an inflammatory skin disease. In an aspect, the biomarker according to the present embodiment may be a biomarker for use in predicting the occurrence, predicting the aggravation, and/or determining the severity of acne vulgaris. In an aspect, the biomarker according to the present embodiment may be a biomarker for use in predicting the occurrence of acne vulgaris and/or determining the severity. The biomarker according to the present embodiment, which is a biomarker for use in predicting the occurrence, predicting the aggravation, and/or determining the severity of acne vulgaris, is such that the amount varies with the severity of acne vulgaris. Therefore, the biomarker according to the present embodiment allows determination and prediction of the state such as severity and progression of acne vulgaris by taking the amount of the biomarker as an indicator.

The biomarker according to the present embodiment is preferably a biomarker for use in predicting the occurrence, predicting the aggravation, and/or determining the severity of acne vulgaris, the biomarker being at least one selected from the group consisting of the following (I) to (III):
(I) at least one human gene selected from the group consisting of IL10, RBP4, CXCL8, NOD2, CYP11B1, CCR1, GSTM1, MEN1, DLK1, DPF2, SLC52A2, KRT17, SEC24C, MSX2, ISCU, DPAGT1, LCT, SAG, LFNG, PAPOLG, IL18BP, HOXA13, VIP, MC5R, B3GNT2, CSK, FGF6, AP5B1, and IL17D, and a biomolecule encoded by the at least one human gene;
(II) at least one bacterium selected from the group consisting of Staphylococcus sp., Pseudomonas sp., Lactobacillus sp., Peptoniphilus sp., Brevundimonas sp., Leptotrichia sp., Prevotella sp., Corynebacterium sp., Dialister sp., Capnocytophaga sp., Brevibacterium sp., Massilia sp., Sphingomonas sp., Streptococcus sp., and Lachnospiraceae sp., and a nucleic acid derived from the at least one bacterium; and
(III) at least one pathway selected from the group consisting of pathways of the following derived from Propionibacterium acnes:
   PWY-7220: adenosine deoxyribonucleotides de novo biosynthesis II,
   PWY-7222: guanosine deoxyribonucleotides de novo biosynthesis II,
   PWY-7208: superpathway of pyrimidine nucleobases salvage,
   PWY-7219: adenosine ribonucleotides de novo biosynthesis,
   PWY-7220: adenosine deoxyribonucleotides de novo biosynthesis II,
   PWY-7222: guanosine deoxyribonucleotides de novo biosynthesis II,
   PWY-7228: superpathway of guanosine nucleotides de novo biosynthesis I,
   PWY-6609: adenine and adenosine salvage III,
   PWY-6897 thiamin salvage II,
   PWY-7221: guanosine ribonucleotides de novo biosynthesis,
   DAPLYSINESYN-PWY: L-lysine biosynthesis I,
   PWY-3841: folate transformations II,
   PWY-2942: L-lysine biosynthesis III, and
   COA-PWY: coenzyme A biosynthesis I, and
   pathways of the following derived from Staphylococcus epidermidis:
      PWY-5100: pyruvate fermentation to acetate and lactate II,
      VALSYN-PWY: L-valine biosynthesis, and
      PWY-7111: pyruvate fermentation to isobutanol (engineered),
      at least one biomolecule constituting the at least one pathway, and a nucleic acid encoding the biomolecule.

In an aspect, the biomarker according to the present embodiment may be at least one human gene corresponding to the above-described item (I) or a biomolecule encoded by the at least one human gene, or may be at least one human gene corresponding to the above-described item (I). In one aspect, the biomarker according to the embodiment may be at least one bacterium corresponding to the above-described item (II) or a nucleic acid derived from the at least one bacterium, or may be a nucleic acid derived from at least one bacterium corresponding to the above-described item (II). In one aspect, the biomarker according to the embodiment may be at least one selected from the group consisting of at least one pathway corresponding to the above-described item (III), at least one biomolecule constituting the at least one pathway, and a nucleic acid encoding the biomolecule; may be at least one selected from the group consisting of at least one biomolecule constituting at least one pathway corresponding to the above-described item (III) and a nucleic acid encoding the biomolecule; may be at least one selected from the group consisting of at least one pathway corresponding to the above-described item (III) derived from Propionibacterium acnes or derived from Staphylococcus epidermidis, at least one biomolecule constituting the at least one pathway, and a nucleic acid encoding the biomolecule; or may be at least one selected from the group consisting of at least one biomolecule constituting at least one pathway corresponding to the above-described item (III) derived from Propionibacterium acnes or derived from Staphylococcus epidermidis, and a nucleic acid encoding the biomolecule. In these cases, a biomolecule constituting a pathway may be, for example, an enzyme constituting a pathway.

The present disclosure relates to, for example, the following (A) to (C).
(A) A biomarker for use in diagnosing the development or severity of acne vulgaris, the biomarker being a gene selected from the group consisting of IL10, RBP4, CXCL8, NOD2, CYP11B1, CCR1, GSTM1, MEN1, DLK1, DPF2, SLC52A2, KRT17, SEC24C, MSX2, ISCU, DPAGT1, LCT, SAG, LFNG, PAPOLG, IL18BP, HOXA13, VIP, MC5R, B3GNT2, CSK, FGF6, AP5B1, and IL17D, or a protein or a fragment thereof encoded by the gene.
(B) A biomarker for use in diagnosing the development or severity of acne vulgaris, the biomarker being a bacterium selected from the group consisting of Staphylococcus sp., Pseudomonas sp., Lactobacillus sp., Peptoniphilus sp., Brevundimonas sp., Leptotrichia sp., Prevotella sp., Corynebacterium sp., Dialister sp., Capnocytophaga sp., Brevibacterium sp., Massilia sp., Sphingomonas sp., Streptococcus sp., and Lachnospiraceae sp.
(C) A biomarker for use in diagnosing the development or severity of acne vulgaris, the biomarker being a pathway derived from Propionibacterium acnes or a pathway derived from Staphylococcus epidermidis, in which the pathway derived from Propionibacterium acnes is a pathway selected from the group consisting of:
   PWY-7220: adenosine deoxyribonucleotides de novo biosynthesis II,
   PWY-7222: guanosine deoxyribonucleotides de novo biosynthesis II,
   PWY-7208: superpathway of pyrimidine nucleobases salvage,
   PWY-7219: adenosine ribonucleotides de novo biosynthesis,
   PWY-7220: adenosine deoxyribonucleotides de novo biosynthesis II,
   PWY-7222: guanosine deoxyribonucleotides de novo biosynthesis II,
   PWY-7228: superpathway of guanosine nucleotides de novo biosynthesis I,
   PWY-6609: adenine and adenosine salvage III,
   PWY-6897 thiamin salvage II,
   PWY-7221: guanosine ribonucleotides de novo biosynthesis,
   DAPLYSINESYN-PWY: L-lysine biosynthesis I,
   PWY-3841: folate transformations II,
   PWY-2942: L-lysine biosynthesis III, and
   COA-PWY: coenzyme A biosynthesis I, and
   the pathway derived from Staphylococcus epidermidis is a pathway selected from the group consisting of:
      PWY-5100: pyruvate fermentation to acetate and lactate II,
      VALSYN-PWY: L-valine biosynthesis, and
      PWY-7111: pyruvate fermentation to isobutanol (engineered).

In the present embodiment, IL10 represents Interleukin-10, RBP4 represents Retinol Binding Protein 4, CXCL8 represents C-X-C motif Chemokine Ligand 8 (also known as IL8 or Interleukin-8), NOD2 represents Nucleotide-binding Oligomerization Domain-containing Protein 2, CYP11B1 represents Cytochrome P450 11B1, CCR1 represents C-C Chemokine Receptor Type 1, GSTM1 represents Glutathione S-transferase Mu 1, MEN1 represents a human gene encoding Menin, which is a protein related to Multiple Endocrine Neoplasia Type 1, DLK1 represents Delta Like Non-canonical Notch Ligand 1, DPF2 represents Double PHD Fingers 2, SLC52A2 represents Solute Carrier Family 52 Member 2, KRT17 represents Keratin 17, SEC24C represents SEC 24 homolog C, MSX2 represents Msh Homeobox 2, ISCU represents Iron-sulfur cluster assembly enzyme, DPAGT1 represents Dolichyl-phosphate N-acetylglucosamine phosphotransferase 1, LCT represents Lactase, SAG represents s-Antigen Visual Arrestin, LFNG represents LFNG O-fucosylpeptide 3-beta-N-acetylglucosaminyltransferase (also known as Spondylocostal dysostosis 3 or SCDO3), PAPOLG represents Poly(A) polymerase gamma, IL18BP represents Interleukin-18-binding protein, HOXA13 represents homeobox A13, VIP represents Vasoactive Intestinal Peptide, MC5R represents Melanocortin 5 Receptor, B3GNT2 represents Beta-1,3-N-acetyl glucosaminyltransferase 2, CSK represents C-terminal Src kinase, FGF6 represents Fibroblast Growth Factor 6, AP5B1 represents Adaptor related Protein Complex 5 Subunit Beta 1, and IL17D represents Interleukin 17D.

In the present embodiment, a pathway is indicated as a pathway code based on MetaCyc (Version 27.1, released on August 28, 2023), which is a database storing extensive information on metabolic pathways and enzymes in living organisms.

A subject for the biomarker according to the present embodiment is preferably a mammal, more preferably a human, and even more preferably a biological female human.

The biomarker according to the present embodiment may be a biomarker contained in a sample derived from a subject, or may be a biomarker extracted from a sample derived from a subject or a biomarker contained in such an extract. The sample may be, for example, a swab sample, sebum, pus, or blood, and in a preferred embodiment, the sample may be a swab sample. The swab sample in these cases may be, for example, a swab sample collected from the skin or mucosa, and in a preferred aspect, the swab sample may be one collected from the skin (skin swab sample). Furthermore, a sample derived from a subject may be a sample obtained by appropriately preparing a subject specimen collected from a subject according to a measurement method. In the present disclosure, a swab sample means a sample collected by bringing a swab into contact with (for example, gently rubbing against) the surface of a site (for example, skin or mucosa) of a target from which the sample is collected.

In the present disclosure, the "amount" of a biomarker is not particularly limited as long as it is a parameter that is an index of the amount of the biomarker present, and may be, for example, concentration (molar concentration, mass per unit volume, or number per unit volume), signal intensity (absorbance, fluorescence intensity, or mass intensity), or the like, and the signal intensity may be a signal intensity derived from a labelling agent that labels a target whose amount is measured. Furthermore, when the biomarker is a human gene or a nucleic acid, the amount of the biomarker may be measured by a method of using a next-generation sequencer or the like, or may be evaluated by a quantitative PCR method based on the amount of an amplification product obtained by amplifying the human gene or nucleic acid by a PCR method, and in the quantitative PCR method, universal primers may be used for the amplification of the nucleic acid. Regarding the method for measuring the amount, the amount is measured based on the method in the Examples.

With regard to the biomarker according to the present embodiment, an increase in the amount of the biomarker in a subject specimen (for example, a skin swab sample) may indicate an increase in the severity (worsening of symptoms) of acne in the subject, or improvement in the likelihood of the development and aggravation of future acne. For example, in a case where the amount of the above-described biomarker in a subject is significantly large as compared with a control subject who is not affected by acne, this may show that the subject has developed acne or there is likelihood of the development of acne. Furthermore, for example, in a case where the amount of the above-described biomarker in a subject is significantly large as compared with a control subject who is affected by non-severe acne vulgaris, this may show that the subject is affected by more severe acne vulgaris than the above-described control subject or it is likely that acne vulgaris may become more severe than the control subject. Furthermore, for example, in a case where the amount of the above-described biomarker in a subject is measured at a plurality of time points, when a time-dependent increase in the amount of the above-described biomarker in a subject is observed, this may show that acne vulgaris in the subject may be aggravated or there is likelihood of aggravation. The biomarker in these cases may be at least one selected from the group consisting of a biomarker corresponding to the above-described item (I), at least one bacterium selected from the group consisting of (II) Staphylococcus sp., Lactobacillus sp., Peptoniphilus sp., Brevundimonas sp., Leptotrichia sp., Prevotella sp., Dialister sp., Capnocytophaga sp., Brevibacterium sp., Massilia sp., Streptococcus sp., and Lachnospiraceae sp., and a nucleic acid derived from the above-described at least one bacterium, and a biomarker corresponding to the above-described item (I). On the other hand, in a case where the biomarker according to an aspect is at least one bacterium selected from the group consisting of Pseudomonas sp., Corynebacterium sp., and Sphingomonas sp., and a nucleic acid derived from the above-described at least one bacterium, for example, a decrease in the amount of the above-described biomarker in a subject specimen (for example, a skin swab sample) may indicate an increase in the severity (worsening of symptoms) of acne in a subject or improvement in the likelihood of the development and aggravation of future acne.

With regard to the biomarker according to the present embodiment, a decrease in the amount of the above-described biomarker in a subject specimen (for example, a skin swab sample) may indicate a decrease in the severity (improvement of symptoms) of acne in a subject, or a decrease in the likelihood of the development and aggravation of future acne. For example, in a case where the amount of the above-described biomarker in a subject is significantly small as compared with a control subject who is not affected by acne, this may show that the subject does not develop acne or has low likelihood of developing acne. Furthermore, for example, in a case where the amount of the above-described biomarker in a subject is significantly small as compared with a control subject who is affected by non-severe acne vulgaris, this may show that the subject is affected by a milder form of acne vulgaris than the above-described control subject or has higher likelihood of having improved acne vulgaris than the control subject. Furthermore, for example, in a case where the amount of the above-described biomarker in a subject is measured at a plurality of time points, when a time-dependent decrease in the amount of the above-described biomarker in a subject is observed, this may show that acne vulgaris in the subject is improved or is likely to be improved. In addition, improvement of acne vulgaris means suppression and reduction of aggravation, suppression of the occurrence of skin rash, and the like. The biomarker in these cases may be, for example, at least one selected from the group consisting of a biomarker corresponding to the above-described item (I), at least one bacterium selected from the group consisting of (II) Staphylococcus sp., Lactobacillus sp., Peptoniphilus sp., Brevundimonas sp., Leptotrichia sp., Prevotella sp., Dialister sp., Capnocytophaga sp., Brevibacterium sp., Massilia sp., Streptococcus sp., and Lachnospiraceae sp., and a nucleic acid derived from the at least one bacterium, and a biomarker corresponding to the above-described item (I). On the other hand, in a case where the biomarker according to an aspect is at least one bacterium selected from the group consisting of Pseudomonas sp., Corynebacterium sp., and Sphingomonas sp., and a nucleic acid derived from the at least one bacterium, for example, an increase in the amount of the above-described biomarker in a subject specimen (for example, a skin swab sample) may indicate a decrease in the severity (improvement of symptoms) of acne in the subject, or a decrease in the likelihood of the development and aggravation of future acne.

With regard to the biomarker according to the present embodiment, since the expression level varies significantly near the surface layer of the skin in patients with high-severity acne vulgaris, it is considered that it is possible to determine the severity of acne or estimate the likelihood of development in the future.

Since the biomarker of the present embodiment not only allows determination or prediction concerning the severity of acne vulgaris, but also allows estimation of the development and aggravation of acne, or checking of the current skin condition (homeostasis) regardless of the presence or absence of the development of acne, it is possible to predict the likelihood of the occurrence of future acne, and without being limited to acne, application as a tool to help prevention of the affection or aggravation of inflammatory skin diseases can be expected.

Furthermore, by carrying out determination using the biomarker according to the present embodiment before and after the application of influencing factors such as administration of a specific component or composition, treatment, lifestyle habits, and diet, changes in the skin symptoms (improvement, worsening, no effect, or the like) caused by the influencing factors can be determined or predicted.

### [Determination or prediction of development or severity of acne vulgaris]

An aspect of the present embodiment relates to a method for determining severity of acne vulgaris in a subject, the method including measuring the biomarker according to an aspect of the present embodiment. An aspect of the present embodiment relates to a method for assisting in the determination of the severity of acne vulgaris in a subject, the method including measuring the biomarker according to an aspect of the present embodiment. An aspect of the present embodiment relates to a method for collecting data for determining the severity of acne vulgaris in a subject, the method including measuring the biomarker according to an aspect of the present embodiment. The biomarker, subject, sample, measurement of the amount, and the determination of severity based on the measured amount are similar to those described for the biomarker according to an aspect of the present embodiment.

An aspect of the present embodiment relates to a method for predicting the likelihood of the occurrence or the likelihood of aggravation of acne vulgaris in a subject, the method including measuring the biomarker according to an aspect of the present embodiment. An aspect of the present embodiment relates to a method for assisting in the prediction of the likelihood of the occurrence or the likelihood of aggravation of acne vulgaris in a subject, the method including measuring the biomarker according to an aspect of the present embodiment. An aspect of the present embodiment relates to a method for predicting the likelihood of the occurrence of inflammation in the skin, the method including measuring the biomarker according to an aspect of the present embodiment. Low likelihood of the occurrence of inflammation in the skin can be said otherwise to mean that homeostasis is maintained, and the condition is satisfactory. An aspect of the present embodiment relates to a method for collecting data for predicting likelihood of occurrence or likelihood of aggravation of acne vulgaris in a subject, the method including measuring the biomarker according to an aspect of the present embodiment. The biomarker, subject, sample, measurement of the amount, and the prediction of the likelihood of occurrence or the likelihood of aggravation based on the measured amount are similar to those described for the biomarker according to an aspect of the present embodiment.

### [Screening methods]

An aspect of the present embodiment is a screening method for a substance or composition that suppresses the expression of a biomarker for predicting the occurrence and/or determining the severity of acne vulgaris; a screening method for a skin microbiome-improving substance, a skin microbiome-improving composition, or a substance or a composition for treating, improving, alleviating, or preventing an inflammatory skin disease (for example, acne vulgaris); or a screening method for a substance or composition for maintaining a condition in which inflammation is less likely to occur in the skin (for example, a condition in which homeostasis is maintained), the screening method including measuring the biomarker according to an aspect of the present embodiment.

An aspect of the present embodiment may be a screening method for a skin microbiome-improving substance, a skin microbiome-improving composition, or a substance or composition for treating, improving, alleviating, or preventing an inflammatory skin disease (for example, acne vulgaris), the method including measuring the biomarker according to an aspect of the present embodiment.

Regarding these, the inflammatory skin disease is preferably acne vulgaris. According to these screening methods, for example, a substance or composition that suppresses the expression level of a gene whose expression is increased in the skin microbiome or an area affected by an inflammatory skin disease (for example, acne vulgaris), or a biomolecule encoded by the gene, can be selected. According to these screening methods, for example, a substance or composition that increases (complements) the expression level of a gene whose expression is reduced in the skin microbiome or an area affected by an inflammatory skin disease (for example, acne vulgaris), or a biomolecule encoded by the gene, can be selected. According to these screening methods, for example, a substance or composition that contributes to a bacterium that contributes to skin homeostasis (for example, regulating gene expression in the bacterium) can be selected. According to these screening methods, for example, a substance or composition that suppresses the function of a bacterium involved in worsening of a skin disease can be selected.

The biomarker, sample, measurement of the amount, and the determination of severity based on the measured amount are similar to those described for the biomarker according to an aspect of the present embodiment. In the screening methods according to an aspect, in a case where the severity of an inflammatory skin disease (for example, acne vulgaris) is decreased by administration of a test substance or a test composition, the test substance or the test composition can be selected as a skin microbiome-improving substance, a skin microbiome-improving composition, or a candidate thereof, or a substance or composition or a candidate thereof for treating, improving, alleviating, or preventing an inflammatory skin disease (for example, acne vulgaris). For example, in a case where the amount of the biomarker in a subject administered with a test substance or a test composition is smaller than the lower limit of the 68% confidence interval or the 95% confidence interval for the amount of the biomarker in a control subject who is not administered with the test substance or the test composition, the test substance or test composition may be selected as a skin microbiome-improving substance, a skin microbiome-improving composition, or a candidate thereof, and the biomarker in this case may be at least one selected from the group consisting of, for example, a biomarker corresponding to the above-described item (I); (II) at least one bacterium selected from the group consisting of Staphylococcus sp., Lactobacillus sp., Peptoniphilus sp., Brevundimonas sp., Leptotrichia sp., Prevotella sp., Dialister sp., Capnocytophaga sp., Brevibacterium sp., Massilia sp., Streptococcus sp., and Lachnospiraceae sp., and a nucleic acid derived from the at least one bacterium; and a biomarker corresponding to the above-described item (I). Furthermore, in a case where the biomarker according to an aspect is at least one bacterium selected from the group consisting of Pseudomonas sp., Corynebacterium sp., and Sphingomonas sp., and a nucleic acid derived from the at least one bacterium, for example, when the amount of the biomarker in a subject administered with a test substance or a test composition is larger than the upper limit of the 68% confidence interval or the 95% confidence interval for the amount of the biomarker in a control subject who is not administered with the test substance or the test composition, the test substance or test composition may be selected as a skin microbiome-improving substance, a skin microbiome-improving composition, or a candidate thereof.

An aspect of the screening method of the present embodiment may be a screening method for a skin microbiome-improving substance, a skin microbiome-improving composition, or a substance or composition for treating, improving, alleviating, or preventing an inflammatory skin disease (for example, acne vulgaris), the method including measuring the amount of a biomarker according to an aspect of the present embodiment in samples derived from a subject and collected before administration and after administration of the test substance or the test composition to the subject. In this case, when the amount of the biomarker after administration of a test substance or test composition is reduced as compared with the amount before administration, the test substance or test composition may be selected as a skin microbiome-improving substance, a skin microbiome-improving composition, or a candidate thereof, or a substance or composition or a candidate thereof for treating, improving, alleviating, or preventing an inflammatory skin disease (for example, acne vulgaris). For example, in a case where the amount of the biomarker in a sample collected in a second collecting step that will be described below is smaller than the amount collected in a first collecting step that will be described below, the test substance or test composition may be selected as a skin microbiome-improving substance, a skin microbiome-improving composition, or a candidate thereof, or a substance or composition or a candidate thereof for treating improving, alleviating, or preventing an inflammatory skin disease (for example, acne vulgaris).

The subject in the screening methods of the present embodiment may be a human, may be a non-human animal, may be preferably a non-human mammal, and may be, for example, a mouse, a rat, a hamster, or a guinea pig. These subjects may be female. Furthermore, it is preferable that these subjects are affected by an inflammatory skin disease, and it is more preferable that the subjects are affected by acne vulgaris.

Examples of the inflammatory skin disease according to the present embodiment include acne vulgaris, atopic dermatitis, seborrheic dermatitis, folliculitis, psoriasis vulgaris, cutaneous candidiasis, and contract dermatitis, and the inflammatory skin disease is preferably acne vulgaris.

The test substance or test composition in the screening methods of the present embodiment is not particularly limited, and may be a substance or composition known to have the potential for having a skin microbiome-improving effect, or may be a substance or composition that is not known to have such potential. Furthermore, the type of the test substance or test composition is not particularly limited, and may be, for example, an organic small molecule, a salt thereof, or a solvate of the organic small molecule or the salt, may be a nucleic acid, may be a peptide or a protein, or may be a composition containing those. Furthermore, when the subject is a human, the test substance is a substance or composition that has been shown to be safe for humans, and may be, for example, a substance or composition that has been approved as an ingredient for a medicine, a cosmetic, or food, or may be a substance or composition known to have a therapeutic effect or prophylactic effect for a skin disease.

In an aspect, the screening methods of the present embodiment may further include collecting a sample from a subject (first collecting step), administering a test substance or a test composition to the subject (administering step), and collecting a sample from the subject (second collecting step), in this order.

The method for collecting a sample from a subject in the first collecting step and the second collecting step is not particularly limited, and can be carried out by a method that is usually carried out by those skilled in the art in accordance with the type of the sample. For example, when the sample is a swab sample, the method for collecting may be a method of bringing a swab into contact with the collecting site.

The method for administering a test substance or a test composition in the administering step is not particularly limited, and may be, for example, transdermal administration, oral administration, intravenous administration, subcutaneous administration, intramuscular administration, intraperitoneal administration, ophthalmic administration, or nasal administration. Furthermore, the amount of administration of the test substance or test composition in the administering step is also not limited, and may be, for example, 1 ng to 10 g per 1 kg of body weight of the subject.

The time period between the first collecting step and the administering step is not particularly limited, and may be, for example, 1 minute or more or 1 day or more, and may be 30 days or less or 7 days or less. Furthermore, the time period between the administering step and the second collecting step is not particularly limited; however, from the viewpoint of carrying out the second collecting step while an effect of improvement by an administered drug is occurring, the time period may be, for example, 1 day or more, 2 days or more, 3 days or more, 7 days or more, or 14 days or more, and may be 60 days or less, 30 days or less, 15 days or less, 10 days or less, 6 days or less, or 4 days or less.

According to the screening methods of the present embodiment, a skin microbiome-improving substance, a skin microbiome-improving composition, or a candidate thereof, or a substance or composition or a candidate thereof for treating, improving, alleviating, or preventing an inflammatory skin disease (for example, acne vulgaris) can be selected. The substance, the skin microbiome-improving composition, or a candidate thereof selected in this manner can be utilized as, for example, an active ingredient and a candidate thereof of a therapeutic agent, an improving agent, an alleviating agent, or a prophylactic agent for inflammatory skin diseases including acne vulgaris.

### EXAMPLES

Hereinafter, the present invention will be specifically described based on Test Examples; however, the present invention is not intended to be limited to these.

### <Implementation of clinical tests>

For an observational study, fifty women (ten healthy individuals and forty acne patients) aged between 20 to 40 years of age were recruited. The criteria for subject selection are as follows.
1) Healthy women aged 20 or more years and less than 40 years at the time point of obtaining consent
2) Individuals who fall into either A) or B) shown below
   A) Healthy skin (individuals with no skin diseases, including acne vulgaris, on the face)
   B) Individuals with acne vulgaris on the face (individuals having 5 or more rashes on the forehead and 10 or more rashes on the entire face, with no imbalance in the number of rashes between the left side and the right side; individuals with 5 or more rashes diagnosed as inflammatory skin rashes are defined as a severe group)
3) Individuals who can receive full explanation on the purpose and contents of the present study, are capable of consenting, can voluntarily volunteer to participate after fully understanding the purpose and contents, and can agree to participate in the present study in writing
4) Individuals who are able to attend the study on a designated examination date and undergo the examination

The criteria for subject exclusion were as follows.
1) Individuals who are currently being medicated with medicines prescribed by a medical institution
2) Individuals with acne conglobata, acne fulminans, and secondary acne (chlorine-induced, drug-induced acne, and the like)
3) Individuals with facial trauma or keloid lesions
4) Individuals with chronic skin diseases other than acne vulgaris, such as atopic dermatitis
5) Individuals who fall under any of the following:
   A) Use of external preparations (medicines, quasi-drugs, cosmetics, and the like)
      I. Individuals who have used the following preparations within 4 weeks from the examination date
         · Preparations containing retinoids (including adapalene and vitamin A preparations)
      II. Individuals who have used the following preparations within 2 weeks from the examination date
         · Acne treating agents such as antibacterial agents and anti-inflammatory agents
         · Preparations containing benzoyl peroxide
         · Corticosteroids
         · Products that are believed to have a peeling effect on the stratum corneum, such as those containing hydroxy acids (glycolic acid, salicylic acid, lactic acid, and the like) (excluding cleansers)
   B) Procedural treatments
      I. Individuals who have undergone the following procedural treatments within 4 weeks from the examination date
         · Chemical peeling, laser treatment, phototherapy, esthetic treatments, and facial surgery
   C) Medications and the like that act on the whole body (medications intended to have a systemic effect, such as oral administration and injection)
      I. Individuals who have used the following preparations within 12 weeks from the examination date
         · Preparations containing retinoids (including adapalene and vitamin A preparations)
      II. Individuals who have used the following preparations within 4 weeks from the examination date
         · Acne treating agents such as antibacterial agents and anti-inflammatory agents
         · Hormone preparations (including low-dose birth control pills and corticosteroids)
         · Chinese herbal medicines that are believed to be effective against acne (Seijoubofuto, Keigairengyoto, Jumihaidokuto, Tokishakuyakusan, and the like)
      III. Individuals who have used the following preparations within 2 weeks from the examination date
         · Corticosteroids
      IV. Individuals who have used the following preparations within 1 week from the examination date
         All medicines other than I to III
6) Individuals who have the habit of taking or applying drugs for the purpose of treating diseases
7) Individuals with a history or current illness of serious disorders of the liver, kidneys, heart, lungs, blood, and the like
8) Individuals with mental disorders
9) Individuals with coexisting diseases and a history of serious diseases in the digestive organs
10) Individuals who are pregnant, breastfeeding, or possibly pregnant
11) Individuals whose daily alcohol intake exceeds an average of 60 g/day in terms of pure alcohol
12) Individuals who are currently participating in other human clinical tests, or individuals who have participated in other human clinical tests within three months before the examination date
13) Individuals who are additionally determined by the principal research doctor or co-research doctors to be unsuitable to be subjects of the present study

The following items were set as subject management items.
1. Physical activity
   · On the day of examination, subjects are prohibited from exercising and performing any physical activity that is likely to cause sweating until the examination is completed.
   · Subjects should have enough sleep the day before each examination.
2. Bathing and face washing
   · Subjects are prohibited from bathing in hot springs, bathing in medicinal baths, utilization of saunas, and the like for three days before the examination date (normal bathing is permitted).
   · The day before the examination, subjects perform bathing and thorough washing of the face, and then are prohibited from bathing (including showering) and face washing until the end of the examination on the day of examination.
3. Make-up and the like
   · Skin care (use of basic cosmetics) after bathing and face washing the day before examination is allowed; however, thereafter, subjects are prohibited from performing skin care (including basic cosmetics) and make-up until the end of the examination on the day of examination.
4. Any additional actions that are believed to affect the results of the study are prohibited.

The present clinical test was conducted through TES Holdings Co., Ltd., a third-party organization. Furthermore, the present test was conducted under the approval of the ROHTO Pharmaceutical Clinical Research Ethical Review Board and the Ethical Review Board of Osaka University Hospital.

### <Experimental methods>

### Swab sampling method:

The central area of the forehead was rubbed about 10 to 20 times with a swab soaked in a buffer solution to collect a sample, and the sample was stored at -20°C or lower.

### Microbiome analysis method:

DNA was extracted from the swabs by using E.Z.N.A.TM stool DNA isolation kit (Omega Bio-Tek). DNA samples were prepared into a library using KAPA Hyper Plus Library Preparation Kit. Universal primers (F515 and R806 (Kozich et al., 2013)) that extend the 16S rRNA v4 region were used as analysis primers, and sequencing analysis was performed using MiSeq. Raw data was subjected to OUT clustering (> 97%) using Mothur, and taxonomy assignment of metagenomic data was carried out using SILVA. For a comparison of the two groups between a severe acne group (number of inflammatory skin rashes ≥ 5) and a non-severe acne group (number of inflammatory skin rashes < 5), analysis using GENE-E (http://www.broadinstitute.org/cancer/software/GENE-E, accessed on October 31, 2023) and LEfSe (Linear discriminant analysis effect size) analysis (Nicola Segata et al., "Metagenomic biomarker discovery and explanation.", Genome Biol. 2011 Jun 24;12(6) :R60) were performed.

### · RNA analysis method:

RNA was extracted from the swabs using Quick-RNA Fecal/Soil Microbe MicroPrep Kit (ZYMO RESEARCH). RNA samples were amplified and prepared into a library using KAPA Stranded RNA-Seq Library Preparation Kit (KAPABIOSYSTEMS), and analysis was performed using HiSeqX. Raw data was subjected to alignment to reference sequences using BBMap and divided into human-derived RNA and environment-derived RNA. Human-derived RNA data and environment-derived RNA data were each subjected to sequence filtering and given with gene annotation information using HiSat2 for the human-derived RNA data and using HuManN2 for the environment-derived RNA data. A comparison between the two groups was performed using GENE-E, and a correlation analysis was performed using Spearman's rank correlation coefficient.

### [Test Example 1] Analysis of the Skin Microbiome in Severe Acne and Mild Acne

In the present Test Example, the abundance ratio of microbiome in the severe acne group and the non-severe acne group was evaluated by performing 16S rRNA amplicon analysis for the microbiome present in the areas where sampling was performed using swabs. Microbiome analysis of the swab samples obtained as described above was performed by the method described in literature (Roberta Caruso et al., "Dynamic and Asymmetric Changes of the Microbial Communities after Cohousing in Laboratory Mice", Cell Rep. 2019 Jun 11;27(11):3401-3412.e3.).

The results are shown in Table 1. A total of 1,614 species of bacteria were detected, and bacteria classified into the genera Staphylococcus, Actinomycetales, Streptococcus, Pseudomonas, or Enhydrobacter accounted for 40% of the total bacteria that we could detect. Through a comparison of two groups between the severe acne group and the non-severe acne group, the microbiome that were significantly more prevalent in the severe acne group were found to be Staphylococcus sp., Lactobacillus sp., Peptoniphilus sp., Brevundimonas sp., Leptotrichia sp., Prevotella sp., , Dialister sp., Capnocytophaga sp., Brevibacterium sp., Massilia sp., Streptococcus sp., and Lachnospiraceae sp., while the microbiome that were significantly more prevalent in the non-severe acne group were found to be Pseudomonas sp., Corynebacterium sp., and Sphingomonas sp., by both the LEfSe and GENE-E analysis methods (p < 0.05, FDR < 0.1).

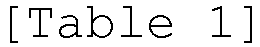

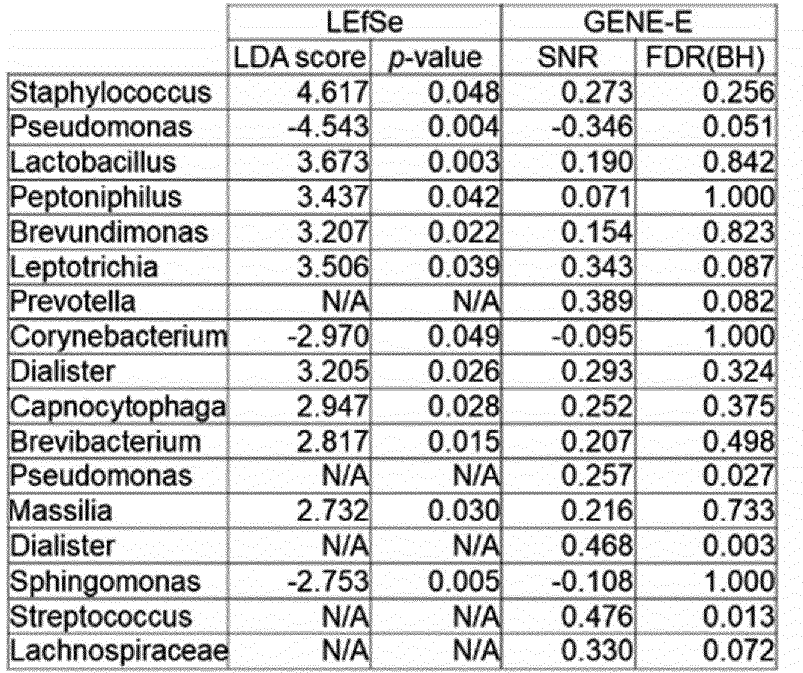

### [Test Example 2] Evaluation of human-derived genes associated with the severity of acne

In the present Test Example, human-derived genes associated with the severity of acne were evaluated.

The results are shown in Table 2. As a result of a two-group comparison analysis by GENE-E, there were 2318 genes that were significantly highly expressed (FDR < 0.05) in the severe acne group compared to the non-severe acne group. There were 1387 genes that were found upon a search for "severe acne" in the public database (GeneCards (www.genecards.org)), and there were 36 genes that were found to be common with the severe acne group. Among these, 29 genes were found by GENE-E as genes that are significantly highly expressed in the severe acne compared to the non-severe acne group (FDR < 0.05), and a significant positive correlation was observed in a correlation analysis using Spearman's rank correlation coefficient. As a result of the present test, human-derived genes associated with the severity of acne were identified.

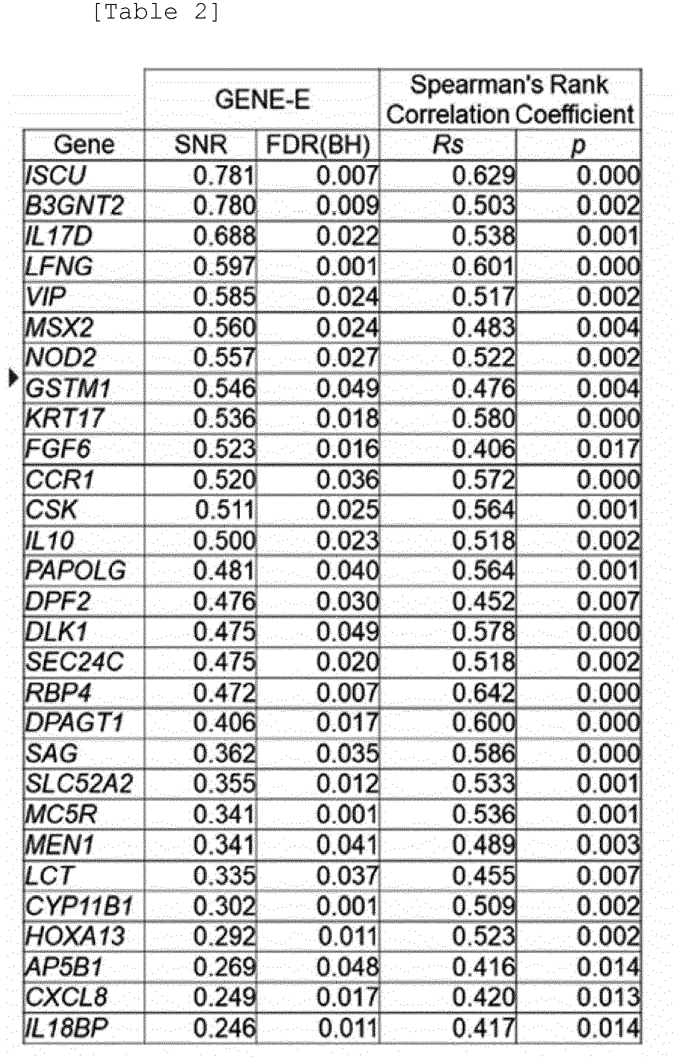

### [Test Example 3] Skin bacterial metatranscriptome analysis

In the present Test Example, the correlation between the severity of acne or the sebum fatty acid content (data not shown) and the bacterial gene pathway (pathway) was examined using Spearman's rank correlation coefficient. Since it was reported that the sebum fatty acid content showed a positive correlation with the severity of acne (Harris HH et al., "Sustainable rates of sebum secretion in acne patients and matched normal control subjects", J Am Acad Dermatol. Volume 8, Issue 2, February 1983, Pages 200-203), the sebum fatty acid content was used as one of the parameters in this metatranscriptome analysis.

The results are shown in Table 3 and Table 4. As a result of analysis using the HUMANn2 platform, a total of 148,177 genes were mapped, and 1,082 pathways were detected. Twelve bacterial pathways shown in Table 3 showed a positive correlation with the severity of acne, but were not correlated with specific bacteria (Rs > 0.4, p < 0.05). Furthermore, there were 16 types of pathways that showed a positive correlation with the amount of fatty acids derived from sebum shown in Table 4, and 11 types of them were derived from C. acnes (Rs > 0.4, p < 0.05).

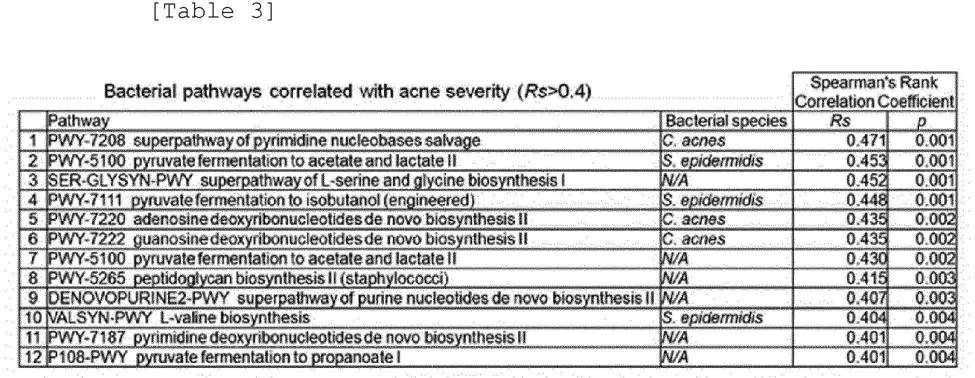

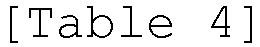

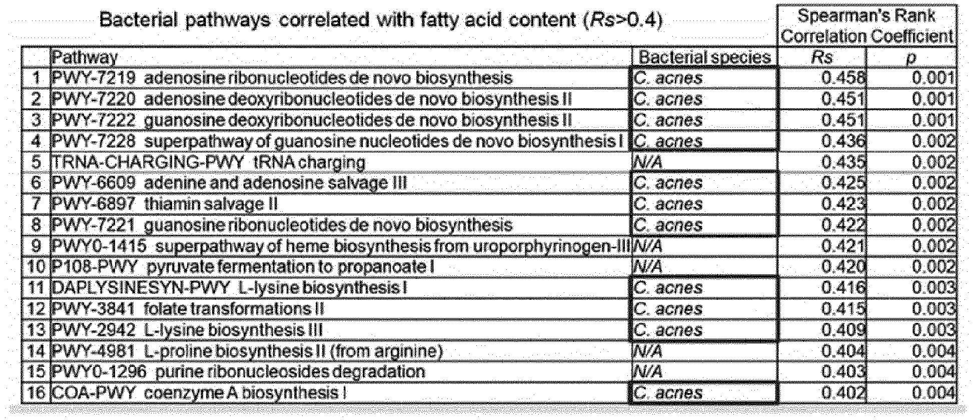

## Claims

1. A biomarker for use in predicting occurrence, predicting aggravation, and/or determining severity of acne vulgaris, the biomarker being selected from the group consisting of the following (I) to (III):
(I) at least one human gene selected from the group consisting of IL10, RBP4, CXCL8, NOD2, CYP11B1, CCR1, GSTM1, MEN1, DLK1, DPF2, SLC52A2, KRT17, SEC24C, MSX2, ISCU, DPAGT1, LCT, SAG, LFNG, PAPOLG, IL18BP, HOXA13, VIP, MC5R, B3GNT2, CSK, FGF6, AP5B1, and IL17D, and a biomolecule encoded by the at least one human gene;
(II) at least one bacterium selected from the group consisting of Staphylococcus sp., Pseudomonas sp., Lactobacillus sp., Peptoniphilus sp., Brevundimonas sp., Leptotrichia sp., Prevotella sp., Corynebacterium sp., Dialister sp., Capnocytophaga sp., Brevibacterium sp., Massilia sp., Sphingomonas sp., Streptococcus sp., and Lachnospiraceae sp., and a nucleic acid derived from the at least one bacterium; and
(III) at least one pathway selected from the group consisting of pathways of the following derived from Propionibacterium acnes:
PWY-7220: adenosine deoxyribonucleotides de novo biosynthesis II,
PWY-7222: guanosine deoxyribonucleotides de novo biosynthesis II,
PWY-7208: superpathway of pyrimidine nucleobases salvage,
PWY-7219: adenosine ribonucleotides de novo biosynthesis, PWY-7220: adenosine deoxyribonucleotides de novo biosynthesis II,
PWY-7222: guanosine deoxyribonucleotides de novo biosynthesis II,
PWY-7228: superpathway of guanosine nucleotides de novo biosynthesis I,
PWY-6609: adenine and adenosine salvage III,
PWY-6897 thiamin salvage II,
PWY-7221: guanosine ribonucleotides de novo biosynthesis,
DAPLYSINESYN-PWY: L-lysine biosynthesis I,
PWY-3841: folate transformations II,
PWY-2942: L-lysine biosynthesis III, and
COA-PWY: coenzyme A biosynthesis I, and
pathways of the following derived from Staphylococcus epidermidis:
PWY-5100: pyruvate fermentation to acetate and lactate II,
VALSYN-PWY: L-valine biosynthesis, and
PWY-7111: pyruvate fermentation to isobutanol (engineered),
at least one biomolecule constituting the at least one pathway, and a nucleic acid encoding the biomolecule.

2. The biomarker according to Claim 1, wherein a subject for the biomarker is a human.

3. The biomarker according to Claim 1 or 2, wherein the biomarker is contained in a swab sample collected from a subject.

4. The biomarker according to Claim 3, wherein the swab sample is collected from the skin.

5. The biomarker according to Claim 1 or 2, wherein an increase in an amount of the biomarker indicates an increase in severity of acne vulgaris in a subject.

6. The biomarker according to Claim 1 or 2, wherein a decrease in an amount of the biomarker indicates a decrease in severity of acne vulgaris in a subject.

7. A method for determining severity of acne vulgaris in a subject, the method comprising:
measuring the biomarker according to Claim 1 or 2 in a sample derived from the subject.

8. A method for assisting in determination of severity of acne vulgaris in a subject, the method comprising:
measuring the biomarker according to Claim 1 or 2 in a sample derived from the subject.

9. A method for collecting data for determining severity of acne vulgaris in a subject, the method comprising:
measuring the biomarker according to Claim 1 or 2 in a sample derived from the subject.

10. A method for predicting likelihood of occurrence or likelihood of aggravation of acne vulgaris in a subject, the method comprising:
measuring the biomarker according to Claim 1 or 2 in a sample derived from the subject.

11. A method for assisting in prediction of likelihood of occurrence or likelihood of aggravation of acne vulgaris in a subject, the method including:
measuring the biomarker according to Claim 1 or 2 in a sample derived from the subject.

12. A method for collecting data for predicting likelihood of occurrence or likelihood of aggravation of acne vulgaris in a subject, the method comprising:
measuring the biomarker according to Claim 1 or 2 in a sample derived from the subject.

13. A screening method for a skin microbiome-improving substance or a skin microbiome-improving composition, the screening method comprising:
measuring the biomarker according to Claim 1 or 2 in a sample derived from the subject.

14. A screening method for a skin microbiome-improving substance or a skin microbiome-improving composition, the screening method comprising:
measuring an amount of the biomarker according to Claim 1 or 2 in samples derived from a subject collected before administration and after administration of a test substance or a test composition to the subject.

15. A screening method for a substance or composition for treating, improving, alleviating, or preventing acne vulgaris, the screening method comprising:
measuring the biomarker according to Claim 1 or 2 in a sample derived from the subject.

16. A screening method for a substance or composition for treating, improving, alleviating, or preventing acne vulgaris, the screening method comprising:
measuring an amount of the biomarker according to Claim 1 or 2 in samples derived from a subject collected before administration and after administration of a test substance or a test composition to the subject.
